(19) Europäisches Patentamt
European Patent Office
Office européen des brevets

(11) Publication number: **0 104 011 B1**

(12) **EUROPEAN PATENT SPECIFICATION**

(45) Date of publication of patent specification: **23.04.86**

(21) Application number: **83304961.2**

(22) Date of filing: **26.08.83**

(51) Int. Cl.[4]: **C 07 D 239/26,** C 07 D 239/34, C 09 K 19/34

(54) **Pyrimidine derivatives.**

(30) Priority: **26.08.82 JP 148283/82**

(43) Date of publication of application: **28.03.84 Bulletin 84/13**

(45) Publication of the grant of the patent: **23.04.86 Bulletin 86/17**

(84) Designated Contracting States: **CH DE GB LI**

(58) References cited:
**FR-A-2 324 636**

**ZEITSCHRIFT FÜR NATURFORSCHUNG, vol. 33b, 1978, pages 433-438**

(73) Proprietor: **Chisso Corporation**
**6-32, Nakanoshima 3-chome Kita-ku**
**Osaka-shi Osaka-fu (JP)**

(72) Inventor: **Fukui, Masahiro**
**2126-5 Kamariyacho Kanazawaku**
**Yokohamashi Kanagawaken (JP)**
Inventor: **Kitano, Kisei**
**10-2 Otsutomocho Kanawaku**
**Yokohamashi Kanagawaken (JP)**
Inventor: **Tanaka, Masami**
**10-3 Otsutomocho Kanazawaku**
**Yokohamashi Kanagawaken (JP)**
Inventor: **Goto, Yasuyuki**
**10-3 Otsutomocho Kanazawaku**
**Yokohamashi Kanagawaken (JP)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 104 011 B1

**Description**

This invention relates to novel organic compounds, and more particularly it relates to novel liquid crystal compounds useful in liquid crystal materials.

As is well known, liquid crystal substances have not only been applied to display elements using nematic liquid crystals having a twisted liquid crystal arrangement (the so-called TN cell), but also have been broadly applied to display elements utilizing the guest-host effect of liquid crystals or mixtures thereof containing a suitable pigment, and furthermore, in DS type display elements utilizing the dynamic scattering effect of liquid crystals, display elements utilizing the cholesteric-nematic phase transition of liquid crystals, DAP type display elements utilizing the electric field-controlling birefringence effect of liquid crystals, etc. At present, however, no single compound exists which satisfies by itself the required characteristics, i.e. liquid crystal temperature range, actuation voltage, response properties, etc, and it is the present state of the art that substances which can stand up to practical use have to a greater or lesser extent been obtained by mixing several kinds of liquid crystal compounds.

The object of the present invention is to provide compounds useful as component in superior, practical and stable liquid crystal compositions.

The present invention resides in 5-substituted-2-(4'-substituted biphenylyl)pyrimidines and 5-substituted-2-[4'-trans-4''-substituted cyclohexyl)phenyl]pyrimidines expressed by the general formula:

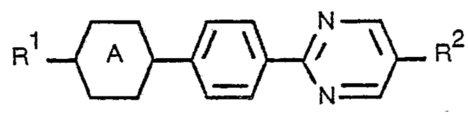

(I)

wherein $R^1$ and $R^2$ each represent an alkyl group or an alkyloxy group having 1 to 10 carbon atoms and the group

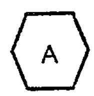

represents a cyclohexane ring or a benzene ring.

The compounds of the formula (I) of the present invention are liquid crystal compounds having a very large anisotropy of refractive index, a lower viscosity for three-ring compounds, a comparatively broad liquid crystal temperature range, and a superior stability.

The compounds of the present invention have good compatibility with other liquid crystal compounds; hence when they are mixed with liquid crystal compounds of one or more kinds, such as those of the biphenyl group, ester group, azoxy group, cyclohexanecarboxylic acid phenyl ester group, phenylcyclohexane group, phenylmetadioxane group, phenylpyrimidine group etc, improvements can be exhibited in various response characteristics, contrast and sharpness and also a broadening of the liquid crystal temperature ranges.

Liquid crystal compounds having a pyridine ring, in particular those having two rings such as compounds expressed by the general formula:

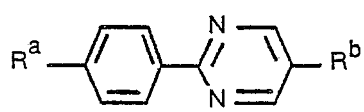

wherein $R^a$ and $R^b$ each represent alkyl, alkoxy, acyl, cyano, etc., are known (see DE 2,257,588, Japanese patent publication No 55—6632 (GB 1,473,990), Japanese patent application laid-open No 55—157,571 (GB 2,049,692), Japanese patent application laid-open No 56—53661 (US 4,311,610) etc). However, such compounds have narrower liquid crystal temperature ranges than those of the present invention.

Liquid crystal compounds in the pyrimidine group having three directly linked rings are known, see the compounds expressed by the general formula:

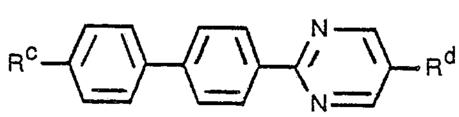

wherein one of $R^c$ and $R^d$ is CN and the other is alkyl, alkyloxy, etc, or by the formula:

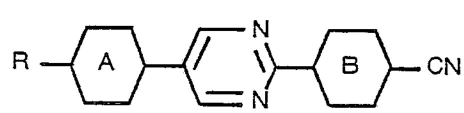

wherein

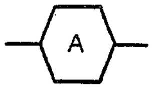 and 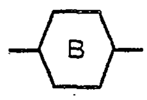 each are 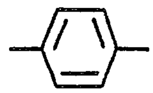 or 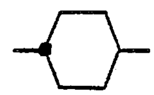

and R is alkyl, etc in Japanese patent publication No 55—27056 USP 4,062,789); Japanese patent application laid-open No 55—104270 (GB 2,042,533, USP 4,273,929); Japanese patent application laid-open No 57—95965 (GB 2,085,877).

However, these compounds necessarily contain a CN group at one end of the molecule, in order to have a large Δε value; hence they have a smaller Δη value, a higher viscosity than those of the compounds of the present invention. Further the abovementioned Japanese patent application No 57—95965 discloses liquid crystal compounds of the pyrimidine group having three directly linked rings such as those expressed by the general formula:

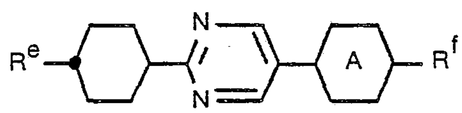

wherein $R^e$ and $R^f$ each represent alkyl, etc, and

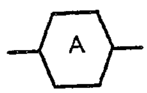 represents 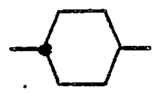 or 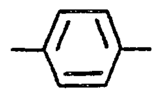

However, if the pyrimidine ring is positioned at the centre of the three rings, two phases (i.e. a smectic phase and a nematic phase) constitute the liquid crystal phase, whereas the compounds of the present invention exhibit only a broad nematic phase; hence they are practically superior.

A preferred preparation of the compounds (I) of the present invention will be described in detail. First, its outline will be illustrated by way of the following equations:

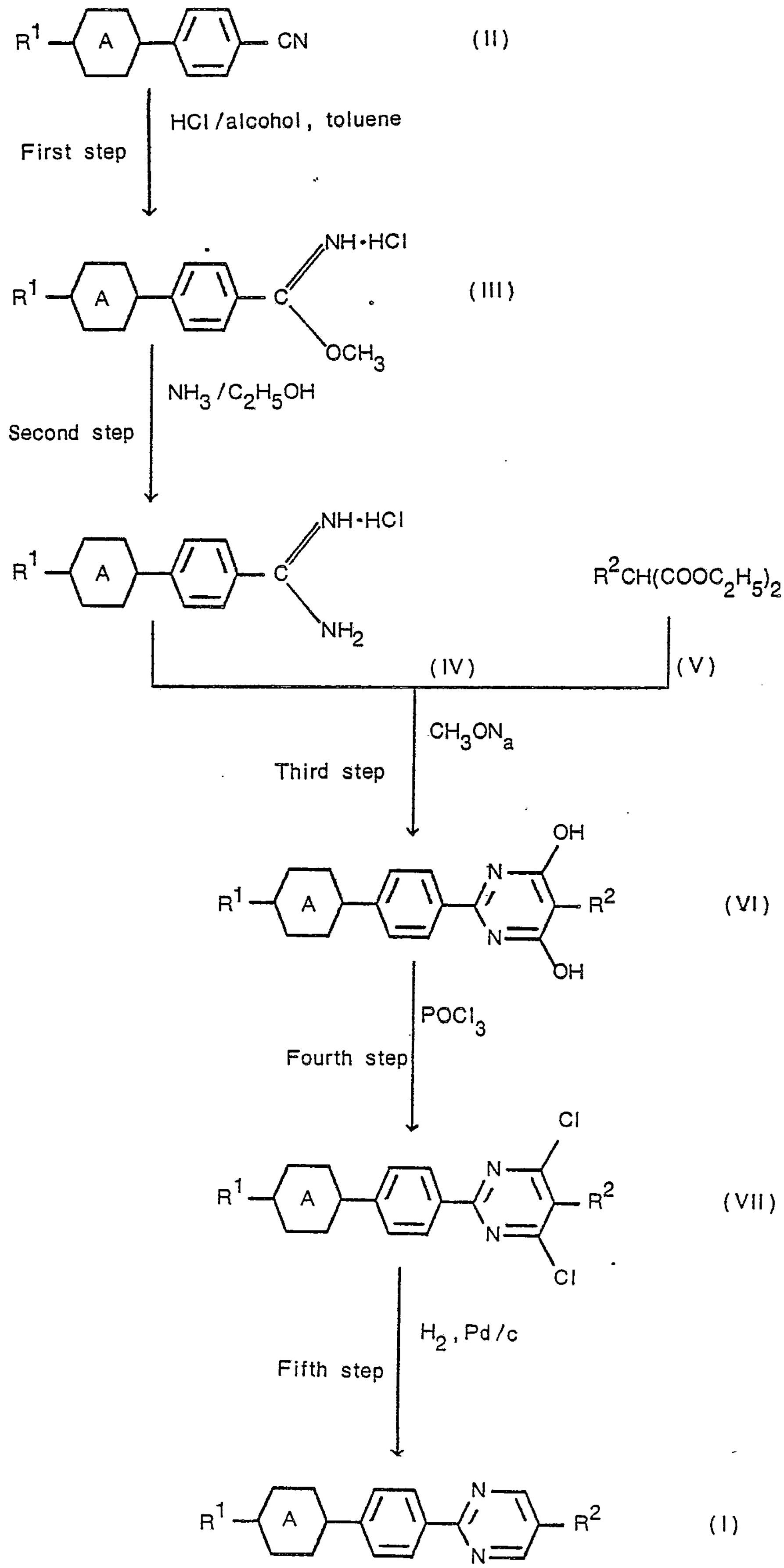
(II)
First step
HCl/alcohol, toluene
(III)
Second step
$NH_3/C_2H_5OH$
(IV)
$R^2CH(COOC_2H_5)_2$
(V)
Third step
$CH_3ONa$
(VI)
Fourth step
$POCl_3$
(VII)
Fifth step
$H_2$, Pd/c
(I)

In this reaction scheme, $R^1$ and $R^2$ have the meanings given above.

First, a compound of the formula (II) having a 4-substituted cyanophenyl group (such compounds being commercially available) is dissolved as starting raw material in an alcohol and toluene and reacted with HCl gas to obtain a compound of the formula (III) i.e. an imide ether hydrochloric acid salt derivative, which is then reacted with an ethanol solution of $NH_3$ to obtain a compound of the formula (IV), i.e. an amidine hydrochloric acid salt derivative, which is then subjected together with a compound of the formula (V) i.e. an alkyl or alkoxymalonic acid diethyl ester, to a cyclization reaction in the presence of sodium methoxide, to obtain a compound of the formula (VI) i.e. a 2,5-substituted-4,6-dihydroxypyrimidine derivative, which is then chlorinated with phosphorous oxychloride to obtain a compound of the formula (VII) i.e. a 2,5-substituted-4,6-dichloropyrimidine derivative, which is then reduced with hydrogen in the presence of a palladium-carbon catalyst to obtain the objective compound (I).

The present invention will be described in detail by way of non-limiting Examples.

Example 1

Preparation of 5-hexyl-2-(4'-pentylbiphenylyl)pyrimidine.

First step

Commercially available 4'-pentyl-4-cyanobiphenyl (102.2 g, 0.410 mol) was dissolved in anhydrous methanol (27.9 g, 0.87 mol) and toluene (100 ml) and the solution was purged by nitrogen gas with stirring at −5°C. HCl gas (16.5 l) was then passed through the solution for 40 minutes and then the reaction mixture was agitated at −5°C for 6 hours.

The reaction mixture was allowed to stand at −20°C for 2 days to deposit crystals, followed by adding ethyl ether (30 ml), filtering and drying the crystals to obtain 4-(4'-pentylphenyl)phenylimide acid methyl ester hydrochloric acid salt (yield 104.5 g, 80%). M P: 231°C.

Second step

Ethanol (300 ml) was added to 4-(4'-pentylphenyl)phenylimide acid methyl ester hydrochloric acid salt (104.5 g, 0.329 mol) obtained at the first step, followed by further adding $NH_3$-ethanol solution (15.9% by weight) (700 ml) with stirring, agitating the mixture at room temperature for 2 hours, filtering, distilling off ethanol from the filtrate, filtering and drying deposited crystals to obtain 4-(4'-pentylphenyl)phenylamidine hydrochloric acid salt (yield: 86.2 g, 96.5%).

Third step

Metal sodium (5.0 g, 0.217 mol) was added to anhydrous methanol (100 ml) to obtain sodium methoxide, to which was then added 4-(4-pentylphenyl)phenylamidine hydrochloric acid salt (20.0 g, 0.066 mol) obtained at the second step and n-hexylmalonic acid diethyl ester (16.2 g, 0.066 mol), under ice cooling and stirring, followed by refluxing with stirring for 8 hours, then ice cooling, adding 20% hydrochloric acid (150 ml), filtering precipitates, washing crystals with water and methanol and drying to obtain 5-hexyl-2-(4'-pentylbiphenylyl)-4,6-dihydroxypyrimidine (yield: 26.2 g, 95%).

Fourth step

To 5-hexyl-2-(4'-pentylbiphenylyl)-4,6-dihydroxypyrimidine (26.2 g, 0.063 mol) obtained at the third step were added phosphorous oxychloride (150 ml) and N,N'-diethylaniline (25 ml), followed by refluxing for 30 hours, then distilling off excess phosphorous oxytrichloride, pouring the reaction mixture while hot into an ice-cooled 10% aqueous solution of NaOH (500 ml), extracting the reaction mixture with toluene (200 ml), washing the toluene layer with 20% hydrochloric acid water, then distilling off toluene. recrystallizing from n-heptane (100 ml) and drying to obtain 5-hexyl-2-(4'-pentylbiphenylyl)-4,6-dichloropyrimidine (yield: 20.5 g, 72%).

Fifth step

To 5-hexyl-2-(4'-pentylbiphenylyl)-4,6-dichloropyrimidine (20.5 g, 0.0453 mol obtained at the fourth step were added magnesium oxide (15.0 g), palladium-carbon (5%) (3.0 g), ethanol (200 ml) and water (15 ml). Hydrogen chloride was then passed through the mixture at room temperature till it was saturated therewith, followed by filtering the reation material, washing the residue with toluene, subjecting the toluene layer and the filtrate to distillation, dissolving the residue in toluene (200 ml), washing the solution with 20% hydrochloric acid and 10% aqueous solution of NaOH, further washing with water till the liquid became neutral, distilling off toluene, and recrystallizing the residue from n-heptane (200 ml) to obtain the objective 5-hexyl-2-(4'-pentylbiphenylyl)pyrimidine (yield: 11.5 g, 66%). This compound had a C—N point of 81°C, a N—I point of 164°C, a viscosity at 20°C $\eta_{20}$ of 36 cp and a $\Delta\eta$ of 0.25. The values of elemental analysis accorded well with the theoretical values as follows:

| Element | Observed values | Theoretical values |
|---|---|---|
| C | 83.8% | 83.89% |
| N | 8.8% | 8.86% |
| H | 7.2% | 7.25% |

Examples 2 to 34

Other compounds of the formula (1) were prepared according to the procedure of Example 1. Their physical properties are shown in Table 1 together with those for the compound of Example 1.

TABLE 1

| Example No. | In formula (I) | | | Values of physical properties | | | | |
|---|---|---|---|---|---|---|---|---|
| | $R_1$ | 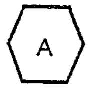 | $R_2$ | C–N point (°C) | N–I point (°C) | $\eta_{20}$ (cp) | $\Delta n$ | $\Delta \epsilon$ |
| 2 | $C_2H_5$ | 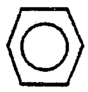 | $C_4H_9$ | 91 | 161 | 30 | 0.27 | 8.5 |
| 3 | ,, | ,, | $C_5H_{11}$ | 105 | 167 | 32 | 0.27 | 9.2 |
| 4 | ,, | ,, | $C_6H_{13}$ | 83 | 155 | 27 | 0.25 | 7.9 |
| 5 | $C_3H_7$ | ,, | $C_2H_5$ | 150 | 179 | 28 | 0.30 | 11.2 |
| 6 | ,, | ,, | $C_4H_9$ | 87 | 175 | 37 | 0.27 | 8.5 |
| 7 | ,, | ,, | $C_6H_{13}$ | 83 | 168 | 36 | 0.25 | 7.9 |
| 8 | $C_5H_{11}$ | ,, | $C_5H_{11}$ | 77 | 173 | 35 | 0.24 | – |
| 1 | ,, | ,, | $C_6H_{13}$ | 81 | 164 | 36 | 0.25 | – |
| 9 | ,, | ,, | $C_7H_{15}$ | 124 | 168 | 37 | 0.27 | – |
| 10 | $C_7H_{15}$ | ,, | $C_3H_7$ | 81 | 168 | 29 | 0.27 | 7.9 |
| 11 | ,, | ,, | $C_4H_9$ | 63 | 157 | 29 | 0.25 | 7.2 |
| 12 | ,, | ,, | $C_6H_{13}$ | 97 | 157 | 35 | 0.24 | 6.5 |
| 13 | $C_2H_5$ | 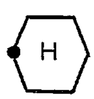 | $C_2H_5$ | 115 | 158 | 43 | 0.19 | 6.5 |
| 14 | ,, | ,, | $C_3H_7$ | 100 | 173 | 30 | 0.19 | 3.9 |
| 15 | ,, | ,, | $C_4H_9$ | 91 | 159 | 48 | 0.17 | 4.5 |

TABLE 1 (Continued)

| Example No. | In formula (I) | | | Values of physical properties | | | | |
|---|---|---|---|---|---|---|---|---|
| | $R_1$ | 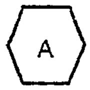 | $R_2$ | C–N point (°C) | N–I point (°C) | $\eta_{20}$ (cp) | $\Delta n$ | $\Delta\epsilon$ |
| 16 | $C_2H_5$ | 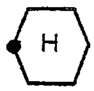 | $C_5H_{11}$ | 91 | 162 | 26 | 0.18 | 3.9 |
| 17 | $C_3H_7$ | ,, | $C_2H_5$ | 109 | 184 | 27 | 0.21 | 6.5 |
| 18 | ,, | ,, | $C_3H_7$ | 97 | 198 | 32 | 0.20 | 5.9 |
| 19 | ,, | ,, | $C_4H_9$ | 79 | 179 | 36 | 0.21 | 3.9 |
| 20 | ,, | ,, | $C_5H_{11}$ | 92 | 184 | 39 | 0.19 | 5.2 |
| 21 | ,, | ,, | $C_6H_{13}$ | 95 | 170 | 41 | 0.15 | 6.2 |
| 22 | ,, | ,, | $C_7H_{15}$ | 106 | 169 | – | – | – |
| 23 | $C_4H_9$ | ,, | $C_2H_5$ | 106 | 174 | 46 | 0.20 | 5.9 |
| 24 | ,, | ,, | $C_3H_7$ | 80 | 187 | 38 | 0.19 | 5.2 |
| 25 | ,, | ,, | $C_4H_9$ | 83 | 172 | 38 | 0.17 | 5.2 |
| 26 | ,, | ,, | $C_5H_{11}$ | 63 | 173 | .30 | 0.16 | 3.9 |
| 27 | $C_5H_{11}$ | ,, | $C_2H_5$ | 116 | 178 | 33 | 0.18 | 6.5 |
| 28 | ,, | ,, | $C_3H_7$ | 101 | 187 | 38 | 0.19 | 5.2 |
| 29 | ,, | ,, | $C_4H_9$ | 79 | 175 | 33 | 0.19 | 4.5 |
| 30 | ,, | ,, | $C_5H_{11}$ | 73 | 177 | 44 | 0.19 | 3.9 |

TABLE 1 (Continued)

| Example No. | In formula (I) | | | Values of physical properties | | | | |
|---|---|---|---|---|---|---|---|---|
| | $R_1$ | 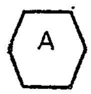 | $R_2$ | C—N point (°C) | N—I point (°C) | $\eta_{20}$ (cp) | $\Delta n$ | $\Delta\epsilon$ |
| 31 | $C_3H_7O$ | 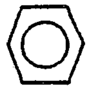 | $C_2H_5$ | 146 | 205 | 56 | 0.32 | — |
| 32 | ,, | ,, | $C_5H_{11}$ | 132 | 204 | — | — | — |
| 33 | ,, | ,, | $C_6H_{13}$ | 123 | 189 | 54 | 0.27 | — |
| 34 | ,, | ,, | $C_7H_{15}$ | 101 | 191 | 47 | 0.25 | 12.2 |

Example 35

A liquid crystal composition A consisting of

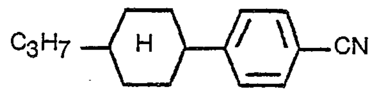

30 parts by weight

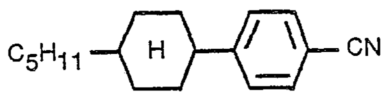

40 parts by weight

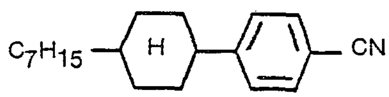

30 parts by weight

had a N—I point of 52.4°C, a viscosity at 20°C $\eta_{20}$ of 23.1 cp and a dielectric anisotropy $\Delta\varepsilon$ of 11.2 ($\varepsilon_{/\!/}$ =16.1, $\varepsilon_{\perp}$ = 4.9). Further, when this composition was sealed in a TN cell of 10 m thick, the resulting threshold voltage, saturation voltage and refractive index anisotropy $\Delta\eta$ were 1.5 V, 2.0 V and 0.120 (ne = 1.612, no = 1.492), respectively.

When the 5-heptyl-2-(4'-pentylbiphenyl)pyrimidine of Example 9 of the present invention (10 parts by weight) was added to the liquid crystal composition A (90 parts by weight), the resulting liquid crystal composition had a N—I point raised up to 61.51°C, a $\eta_{20}$ slightly raised up to 24.5 cp, a $\Delta\varepsilon$ of 11.0 ($\varepsilon_{/\!/}$ = 15.6, $\varepsilon_{\perp}$ = 4.6), a threshold voltage of 1.6 V, a saturation voltage of 2.1 V and a $\Delta\eta$ raised up to 0.135 (ne = 1.629, no = 1.494).

Example 36

5-Ethyl-2-(4'-(trans-4''-propylcyclohexyl)phenyl)pyrimidine (10 parts by weight) of Example 17 of the present invention was added to the same liquid crystal composition A (90 parts by weight) as used in Example 35. The resulting liquid crystal composition had a N—I point raised up to 63.1°C, a $\eta_{20}$ of 24.4 cp similar to that of the composition A, a $\Delta\varepsilon$ of 11.1 ($\varepsilon_{/\!/}$ = 15.7, $\varepsilon_{\perp}$ = 4.6), a threshold voltage of 1.5 V, a saturation voltage of 2.1 V and a $\Delta\eta$ raised up to 0.130 (ne = 1.624, no = 1.494).

Comparative Example

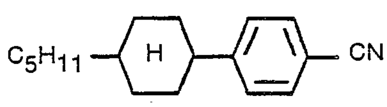

(10 parts by weight)

which is a compound representative of high temperature liquid crystals in current use having a large $\Delta\eta$ value was added to the liquid crystal composition A (90 parts by weight) used in Examples 35 and 36. The

resulting liquid crystal composition had a N—I point raised up to 66.7°C, a Δη raised up to 0.146 and also a $\eta_{20}$ much raised up to 27.5 cp.

Further, when the above mixing proportion was changed to 96.5 parts by weight of the liquid crystal composition A and 3.5 parts by weight of the above high temperature liquid crystal compound, the resulting liquid crystal composition had a $\eta_{20}$ of 24.5 cp, but its N—I poiint and Δη were reduced down to 57.6°C and 0.129, respectively.

**Claims**

1. A 5-substituted-2-(4′-substituted biphenylyl)pyrimidine or 5-substituted-2-[4′-(trans-4″-substituted cyclohexyl)phenyl]pyrimidine expressed by the general formula:

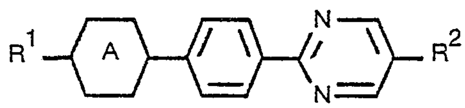 (I)

wherein $R^1$ and $R^2$ each represent an alkyl group or an alkoxy group having 1 to 10 carbon atoms and

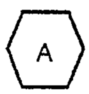

represents a cyclohexane ring or a benzene ring.

2. A 5-substituted-2-[4′-(trans-4″-alkylcyclohexyl)phenyl]pyrimidine expressed by the general formula:

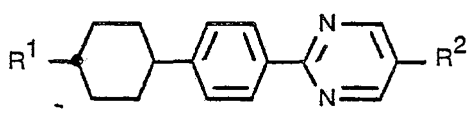

wherein $R^1$ and $^2$ each represent an alkyl group or an alkoxy group having 1 to 10 carbon atoms.

3. A compound of claim 2 wherein $R^1$ and $R^2$ each have 2 to 7 carbon atoms.

4. A compound of claim 2 or 3 wherein $R^1$ and $R^2$ each represent an alkyl group.

5. A liquid crystal composition containing as at least one component thereof, one or more 5-substituted-2-(4′-substituted biphenyl)pyrimidines or 5-substituted-2-[4′-)trans-4″-substituted, cyclohexyl)phenyl]pyrimidines as defined in any preceding claim.

6. A method of preparing a 5-substituted-2-(4′-substituted biphenylyl)pyrimidine or 5-substituted-2-[4′-(trans-4″-substituted cyclohexyl)phenyl]pyrimidine as defined in claim 1, which comprises reductive dehalogenation of a corresponding 4,6-dihalopyrimidine.

**Patentansprüche**

1. 5-substituiertes-2-(4′-substituiertes Biphenylyl)pyrimidin oder 5-substituiertes-2-[4′-(trans-4″-substituiertes Cyclohexyl)phenyl]pyrimidin, das durch folgende allgemeine Formel wiedergegeben wird:

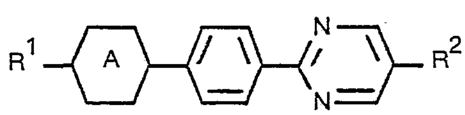 (I)

worin $R^1$ und $R^2$ jeweils eine Alkylgruppe oder eine Alkoxygruppe mit 1 bis 10 Kohlenstoffatomen darstellen und

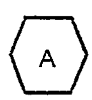

einen Cyclohexanring oder einen Benzolring darstellt.

2. 5-substituiertes -2-[4′-(trans-4″-Alkylcyclohexyl)phenyl]pyrimidin, das durch folgende Formel wiedergegeben wird:

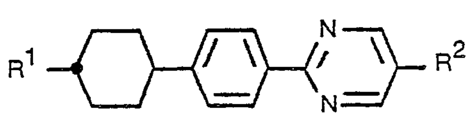

worin $R^1$ und $R^2$ jeweils eine Alkylgruppe oder eine Alkoxygruppe mit 1 bis 10 Kohlenstoffatomen darstellen.

3. Verbindung nach Anspruch 2, worin $R^1$ und $R^2$ jeweils 2 bis 7 Kohlenstoffatome aufweisen.

4. Verbindung nach Anspruch 2 oder 3, worin $R^1$ und $R^2$ jeweils eine Alkylgruppe darstellen.

5. Flüssigkristall-Zusammensetzung, die als wenigstens eine Komponente ein oder mehrere 5-substituierte-2-(4'-substituierte Biphenyl)pyrimidine oder 5-substituierte-2[4'-(trans-4"-substituierte Cyclohexyl)phenyl] pyrimidine nach einem der vorstehenden Ansprüche enthält.

6. Verfahren zur Herstellung eines 5-substituierten-2-(4'-substituierten Biphenylyl)pyrimidin oder 5-substituierten-2-[4'-trans-4"-substituierten Cyclohexyl)phenyl]pyrimidins nach Anspruch 1, welches eine reduktive Dehalogenierung eines entsprechenden 4,6-Dihalogenpyrimidins umfaßt.

## Revendications

1. 2-biphénylyl (substitué en position 4')-pyrimidine, substituée en position 5, ou 2-[4'-cyclohexyl (substitué en position trans-4")phényl]pyrimidine, substituée en position 5, exprimée par la formule générale:

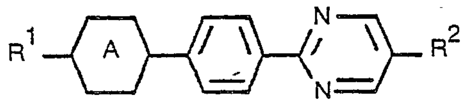

(I)

dans laquelle $R^1$ et $R^2$ représentent chacun un groupe alkyle ou un groupe alcoxy ayant 1 à 10 atomes de carbone et

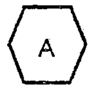

représente un cycle cyclohexane ou un cycle benzène.

2. 2-[4'-(trans-4"-alkylcyclohexyl)-phenyl]pyrimidine, substituée en position 5, exprimée par la formule générale

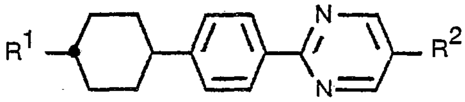

dans laquelle $R^1$ et $R^2$ représentent chacun un groupe alkyle ou un groupe alcoxy ayant 1 à 10 atomes de carbone.

3. Composé selon la revendication 2, dans lequel $R^1$ et $R^2$ ont chacun 2 à 7 atomes de carbone.

4. Composé selon la revendication 2, dans lequel $R^1$ et $R^2$ reprèsentent chacun un groupe alkyle.

5. Composition à cristaux liquides contenant, en tant qu'au moins l'un de ses ingrédients, une ou plusieurs 2-biphenyl (substitué en position 4') pyrimidine, substituée en position 5, ou 2-[4'-cyclohexyl (substitué en position trans-4")phényl] pyrimidine, substituée en position 5, comme défini dans 1'une des revendications précédentes.

6. Méthode de préparation d'une 2-biphénylyl (substitué en position 4')pyrimidine, substituée en position 5, ou 2-[4'-cyclohexyl (substitué en position trans-4") phényl]pyrimidine, substituée en position 5, comme défini dans la revendication 1, qui comprend la déhalogénation réductrice d'une 4,6-dihalogénopyrimidine correspondante.